# EUROPEAN PATENT SPECIFICATION

(11) **EP 1 482 948 B1**
(45) Date of publication and mention of the grant of the patent: **16.08.2006**
(21) Application number: 03716467.0
(22) Date of filing: 11.03.2003
(51) Int. Cl.: A61K 31/565, A61K 31/57, A61P 15/12

(54) **CONTINUOUS SULFATASE INHIBITING PROGESTOGEN HORMONE REPLACEMENT THERAPY**
KONTINUIERLICHE SULFATASE-HEMMENDE PROGESTOGEN ENTHALTENDE HORMONSUBSTITUTIONS-THERAPIE
TRAITEMENT HORMONAL SUBSTITUTIF CONTINU PAR PROGESTOGENE INHIBANT LA SULFATASE

(30) Priority: 11.03.2002 US 363148 P; 17.05.2002 US 381541 P
(43) Date of publication of application: 08.12.2004
(73) Proprietor: JANSSEN PHARMACEUTICA N.V., 2340 Beerse (BE)
(72) Inventor: CAUBEL, Patrick, Michel, Princeton, NJ 08540 (US); FRIEDMEN, Andrew, Joseph, Princeton, NJ 08540 (US)
(74) Representative: Mercer, Christopher Paul
(86) International application number: PCT/US2003/007452
(87) International publication number: WO 2003/077924

(56) References cited:
- EP-A- 0 309 263
- WO-A-00/59577
- WO-A-01/52857
- US-A- 4 906 169
- US-A- 5 968 919
- US-A- 6 071 531
- US-A- 6 165 491

## Description

The present invention relates to the use of hormone formulations in the manufacture of a medicament for use in a particular form of hormone replacement therapy (HRT) for the treatment of post-menopausal or non-menstruating females. More particularly, the medicament for use in that particular form of continuous hormone replacement therapy contains a potent sulfatase inhibiting progestogen that is norgestimate (NGM) or norelgestromin (NGMN), and a suitable estrogen.

### PRIOR ART

Hormone replacement therapy that uses a combination of estrogen and progestogen is in itself known.

WO 00/59577-A discloses a low dose estrogen interrupted hormone replacement therapy in which a pharmaceutical preparation comprises a plurality of doses for consecutive administration doses administered in alternating relatively dominant estrogenic activity phases and a relatively dominant progestogenic activity phases. The relatively dominant estrogenic activity phases comprise three daily doses or an equivalent thereof, of a substance exhibiting estrogenic activity equivalent to about 1 mg per day of 17β-estradiol. The relatively dominant progestogenic activity phases comprise three daily doses or an equivalent thereof, of a combination of a substance exhibiting estrogenic activity equivalent to about 1 mg per day of 17β-estradiol and a substance exhibiting progestogenic activity equivalent to about 90 µg per day of norgestimate.

WO 01/52857-A discloses a pharmaceutical composition for hormone replacement therapy. The composition comprises, as a first active agent, an estrogen (or naturally or synthetic derivative thereof) in sufficient amounts to treat diseases, disorders and symptoms associated with deficient endogenous levels of estrogen in women, and drospirenone as a second active agent in sufficient amounts to protect the endometrium from the adverse effects of estrogen, together with pharmaceutical acceptable excipients or carriers.

US-A-6165491 discloses a composition, based on progesterone and estrogen, which is intended for menopausal hormone replacement therapy. The composition comprises a suspension of progesterone in a lipophilic medium containing 17-estradiol valerate dissolved in said lipophilic medium, in which the weight ratio of progesterone to the corresponding base of 17-estradiol base is 25 to 600.

EP-A-0309263 discloses a contraceptive formulation and its use in a method of contraception and of hormonal replacement. The formulation which employs a combination of estrogen and progestin and wherein a short period of relatively dominant estrogenic activity alternates with a short period of relatively dominant progestagenic activity.

US-A-4906169 discloses transdermal estrogen/progestin absorption dosage units, which comprise a backing layer, an adjoining polymer layer is an adhesive layer in which at least minimum effective dose of an estrogen is dissolved or microdispersed. Adhered to the polymer layer in an adhesive layer in which is dissolved and/or microdispersed at least minimum doses of progestin, preferably the natural estrogen, 17-beta-estradiol, or ethinyl estradiol or combinations thereof and the progestin, norethindrone or norgestimate or combinations thereof. The transdermal absorption dosage units are used in a process of fertility control and estrogen replacement therapy.

US-A-6071531 discloses transdermal drug delivery, in particular patches and methods for transdermally administering 17-deacetyl norgestimate either alone or in combination with an estrogen, particularly ethinyl estradiol. These patches are also can be used to provide hormone replacement therapy.

US-A-5968919 discloses topical alcoholic or aqueous alcoholic gels containing testosterone, progesterone, estradiol or other hormones that have enhanced penetration through skin by including in the formulation 2-n-nonyl-1,3-dioxolane or other hydrocarbyl derivative of 1,3-dioxolane or 1,3-dioxane or acetal, as skin penetration enhancing compound.

### BACKGROUND OF THE INVENTION

A substantial percentage of human breast carcinomas are hormone-dependent. Animal studies and clinical trials have confirmed that estrogens, particularly estradiol, are the most important hormones involved in supporting growth of hormone-dependent breast tumours. (see refs # 1 at 493, #2 at 967, #7 at 1589, #8 at 525, #9 at 135, #10 at 225, #11 at 625 and #12 at 1497).

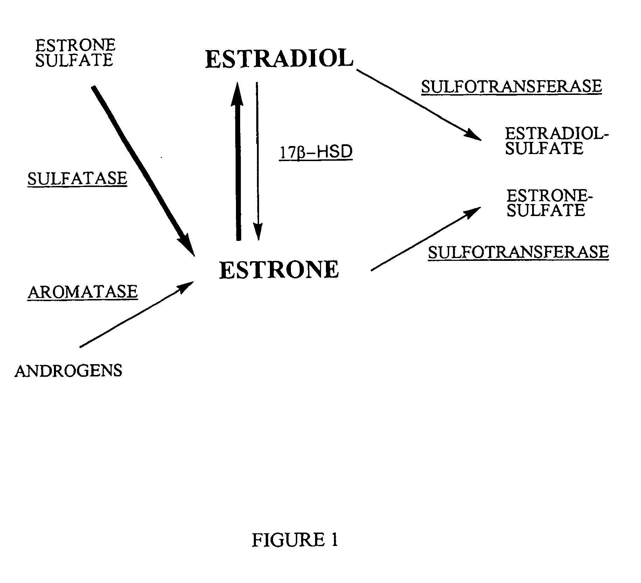

Plasma levels of estrone and estradiol in post-menopausal women are very low. (see refs #1 at 493 and #11 at 626). Yet, breast tumor tissue concentration of estrone and estradiol is an order of magnitude higher than plasma concentrations. (see refs #1 at 493, #2 at 967 and #13 at 641). Figure 1 shows the enzymatic process by which estrogens are locally formed in human breast cancer cells and thereby made available to support growth. (see ref #10 at 229). Referring to Figure 1, studies have shown thatthe sulfatase enzyme appears to be at least 10x more important in the formation of estrogens than the aromatase enzyme. (see refs #1 at 493, #2 at 967, #4 at 17, #5 at 931, #7 at 1589, #8 at 525, #9 at 135, #10 at 228, #11 at 626 and 628 and #13 at 641). Thus, it is the sulfatase pathway that is the primary pathway promoting local formation of estrogens in human breast cancer cells.

Since estradiol is one of the main factors involved in supporting growth of hormone-dependent breast tumours and the sulfatase pathway is the main pathway for the formation of estradiol in the breast, then a decrease of estradiol formation by suppression of the sulfatase pathway would have potential therapeutic activity in the management of breast cancer. (see refs #1 at 493, #3 at 55, #4 at 17, #5 at 931, #6 at 123 and #11 at 631). Suppression of the sulfatase pathway will have a breast protective effect.

In one aspect the present invention provides an HRT regimen to continuously suppress sulfatase activity in human breast cancer cells; or provides an HRT regimen with exceptional suppression of sulfatase activity in human breast cancer cells.

In another aspect the present invention provides an HRT regimen to continuously suppress estrogen formation in human breast cancer cells; or provides an HRT regimen with exceptional suppression of estrogen formation in human breast cancer cells.

In still another aspect the present invention provides an HRT regimen which minimizes exposure of the breast to locally formed estrogen; provides an HRT regimen which reduces exposure of the breast to estrogens, as compared to other HRT regimens of equivalent estrogen dose; or provides an HRT regimen with the lowest levels of breast estrogen exposure as compared to other HRT regimens of equivalent estrogen dose.

In another aspect the present invention provides an HRT regimen which tends to limit exposure of the breast to those levels of estrogen which are administered in the regimen.

In still another aspect the present invention provides an HRT regimen which provides exceptional and continuous breast protective effect.

In another aspect the present invention provides an HRT regimen which minimizes risk factors associated with breast cancer.

### References:

1. Inhibition of Estrone Sulfatase Enzyme in Human Placenta and Human Breast Carcinoma; T.R. JEFFRY EVANS et al., J. Steroid Biochem. Molec. Biol. Vol. 39, No. 4A 1991, pp. 493-499.
2. In Vitro Effect of Synthetic Progestogens on Estrone Sulfatase Activity in Human Breast Carcinoma; ODILE PROST-AVALLET et al., J. Steroid Biochem. Molec. Biol., Vol. 39, No. 6, 1991, pp.967-973.
3. Effect of the progestagen R5020 (promegestone) and of progesterone on the uptake and on the transformation of estrone sulfate in MCF-7 and T-47d human mammary cancer cells: correlation with progesterone receptor levels; JORGE R. PASQUALINI et al., Cancer Letters, 66 (1992) 55-60, Elsevier Scientific Publishers Ireland Ltd.
4. Action of Danazol On The Conversion Of Estrone Sulfate To Estradiol And On The Sulfatase Activity In The MCF-7, T-47D and MDA-MB-231 Human Mammary Cancer Cells; B-L NGUYEN et al., J. Steroid Biochem, Molec. Biol. Vol. 46, No. 1, 1993, pp. 17-23.
5. Effect of Promegestone, Tamoxifen, 4-Hydroxytamoxifen and ICI 164,384 on the Oestrone Sulphatase Activity of Human Breast Cancer Cells; GERARD CHETRITE et al., Anticancer Research 13: 931-934 (1993).
6. Inhibition Of Steroid Sulfatase Activity By Danazol; KJELL CARLSTROM et al., Acta Obstet Gynecol Scand Suppl. 107-111.
7. Effect of the Progestagen Promegestone (R-5020) on mRNA of the Oestrone Sulphatase in the MCF-7 Human Mammary Cancer Cells; JORGE R. PASQUALINI et al., Anticancer Research 14: 1589-1594 (1994).
8. Effect of Nomegestrol Acetate on Estrone-sulfatase and 17β-Hydroxysteroid Dehydrogenase Activities in Human Breast Cancer Cells; G. CHETRITE et al., J. Steroid Biochem. Molec. Biol. Vol. 58, No.5/6, pp. 525-531, 1996.
9. Effect of Tibolone (Org OD14) and its Metabolites on Estrone Sulphatase Activity in MCF-7 and T-47D Mammary Cancer Cells; G. CHETRITE et al., Anticancer Research 17: 135-140 (1997).
10. Progestins and Breast Cancer; J.R. PASQUALINI et al., J. Steroid Biochem. Molec. Biol. Vol. 65, No. 1-6, pp.225-235, 1998.
11. Control of Estradiol In Human Breast Cancer. Effect of Medrogestone on Sulfatase, 17β-Hydroxysteroid Dehydrogenase And Sulfotransferase Activities in Human Breast Cancer Cells; JORGE RAUL PASQUALINI et al., Euro. Congr. On Menopause (1998), pp. 625-633.
12. Constitutive Expression of the Steroid Sulfatase Gene Supports theGrowth of MCF-7 Human Breast Cancer Cells in Vitro and in Vitro; MATTIE R. JAMES et al., Endocrinology, Vol. 142, No.4, pp 1497-1505.
13. Concentrations of Estrone, Estradiol and Their Sulfates, And Evaluation of Sulfatase and Aromatase Activities in Patients with Breast Fibroadenoma; J.R. PASQUALINI et al., Int. J. Cancer, 70, pp. 639-643 (1997).

### SUMMARY OF THE INVENTION

According to the present invention there is provided the use of a combination of estrogen and norgestimate or norelgestromin for the manufacture of a medicament for use in a method of the prevention of treast cancer whilst providing hormone replacement therapy, as defined in the appendant claims.

In one preferred embodiment such therapy comprises a cycle of separate dosage units adapted for successive daily oral administration for the length of the cycle, wherein said dosage units contain, in admixture with a pharmaceutically acceptable carrier, a combination of an estrogen in an effective hormone replacement amount, and norgestimate or norelgestromin in an amount which is both an effective endometrium protective amount and an effective breast protective amount.

In another preferred embodiment such therapy comprises one or more transdermal patches adapted for successive administration for the length of the cycle, wherein said transdermal patches of said unit collectively contain for a cycle of continuous administration a combination of an estrogen in an effective hormone replacement amount, and norgestimate or norelgestromin in an amount which is both an effective endometrium protective amount and an effective breast protective amount.

In a further preferred embodiment such therapy comprises one or more vaginal rings adapted for successive administration for the length of the cycle, wherein said vaginal rings of said unit collectively contain for a cycle of continuous administration a combination of an estrogen in an effective hormone replacement amount, and norgestimate or norelgestromin in an amount which is both an effective endometrium protective amount and an effective breast protective amount.

Applicants have surprisingly discovered that such a regimen is expected to have reduced levels of estrogen in the breast as compared to other hormone replacement therapies having equivalent doses of estrogens.

### BRIEF DESCRIPTION OF THE DRAWINGS

Figure 1 - Shows the enzymatic process involved in the formation and transformation of estrogens in human breast cancers.

### DETAILED DESCRIPTION OF THE INVENTION

The HRT regimen according to the present invention is administered cycle after cycle continuously, without interruption of either estrogen or progestogen administration, to a post-menopausal or estrogen deficient female in need of such therapy. Estrogen deficient females is intended to refer to those women who are prematurely estrogen deficient due to natural or artificial means, such as, chemical castration, removal of the uterus and/or ovaries, etc. The method of administration might be transdermal, vaginal or oral. Where administration is transdermal, a suitable patch is continuously worn with replacement as required. Where administration is vaginal, a suitable vaginal device, such as a ring, is continuously inserted with replacement as required. Where administration is oral, daily oral dosage units are administered.

The cycle of administration usually lasts 28 days or more, but it may be longer up to 60 and even 90 days or shorter down to 21 days. In the case of an HRT regimen, the cycle may include a regimen in which there is a day to day or week to week variation in the dose of active administered according to a set pattern. In such a case the regimen, including variation of dose, is repeated in cycle following cycle. The cycle may also be a regimen in which there is no variation in the dose of the active administered. In such a case, the cycle is nothing more than a convention representing a convenient unit of administration or sale. In either case, an HRT product utilizing the HRT regimen in question is prescribed, sold and administered in units of cycles. The HRT product based on a cycle might be 1 to 10 of vaginal rings that are inserted and then replaced every 7, 14 or 21 days according to their design. The HRT product based on a cycle might be 2 to 10 transdermal patches that are attached and then replaced every 7, 10 or 14 days according to their design. The HRT product based on a cycle might be 21, 28, 56 or more tablets that are orally administered daily.

"Estrogen" herein refers to an estrogen receptor modulator having either an agonistic or antagonistic effect on the estrogen receptor, but preferably an agonistic effect. Any conventional estrogen may be employed as a suitable component in the HRT regimen of this invention. In addition to the commonly employed 17β-estradiol, there can be also be employed 17α-ethinylestradiol, esters and ethers of 17α-ethinylestradiol such as, for example, 17α-ethinylestradiol 3-dimethylamino propionate, 17α-ethinylestradiol 3-cyclopentyl ether (quienestrol) and 17α-ethinylestradiol 3-methyl ether (mestranol) as the estrogen component. Natural estrogens such as estrone, estrone sulfate, estrone sulfate piperazine salt, estradiol and estriol, and their esters, may also be employed. Conjugated equine estrogens (CEE) or conjugated estrogens (CE) are well known for this use. Suitable synthetic estrogens or synthetic estrogen modulators for use herein include tamoxifen, toremifene, ormeloxifene, modrefen, fulvestrant, lasofoxifene, bazedoxifene (TSE-424), arzoxifene, tesmilifene, miproxifene, EM-652 (Sch-57068), 3339 (Aventis), Ospemifene (Fc 1271A), ERA-923, GTx-006, HM-101, DPC-974, A-007, SP-8490, WAY-140424, tibolone, levodoxiphen, raloxifene. The preferred estrogen is 17β-estradiol which is well known to the art of hormone replacement therapy and is now used in commercially available HRT products.

The objective of any HRT regimen is to relieve one or more of several symptoms of menopause or an induced menopause like condition by the administration of sufficient estrogen. The estrogen might be administered in sufficient amounts to relieve hot flushes and night sweats to improve sleep patterns and the patient's general feeling of well-being. The estrogen might be administered in sufficient amounts to protect against loss of calcium from the skeleton, especially from vertebral bodies, preventing crush fractures and loss of body height. The estrogen might be administered in sufficient amount to reduce the risk of death from ischemic heart disease. The estrogen might be administered in sufficient amount to protect the vascularity and health of the vaginal mucosa and urinary tract. Herein the administration of a hormone replacement amount of estrogen to post-menopausal or estrogen deficient females in need of such therapy would provide partial or full relief as to one or more of these objectives.

In one characterization of a hormone replacement amount of estrogen, there is administered by any suitable route sufficient estrogen such that it is equivalent in effect to about 0.25 mg to about 2.5 mg of orally administered 17β-estradiol. Preferably, such estrogen should be administered in an amount that is equivalent in effect to about 0.5 mg to about 1.5 mg of orally administered 17β-estradiol. In another characterization of a hormone replacement amount of estrogen, there is administered by any suitable route sufficient estrogen such that it is equivalent in effect to about 10 mcg to about 200 mcg of orally administered 17α-ethinylestradiol. Preferably, such estrogen should be administered in an amount that is equivalent in effect to about 25 mcg to about 100 mcg of orally administered 17α-ethinylestradiol. It follows that in the case of a daily oral tablet, there is administered a preferred dose of 17β-estradiol (or an HRT equivalent amount of a suitable estrogen) between about 0.25 mg to about 2.5 mg and more preferably between about 0.5 mg to about 1.5 mg. Specific daily oral tablets might contain 0.5, 0.75, 1.0 or 1.5 mg of 17β-mtradiol. In the case of a vaginal ring, the preferred ring delivers to systemic circulation a daily dose of 17β-estradiol (or an HRT equivalent amount of a suitable estrogen) between about 0.15 mg to about 1.5 mg and more preferably between about 0.3 mg to about 0.9 mg. A specific vaginal ring might be inserted for one week and deliver to systemic circulation in that period of time an average daily dose of 0.3, 0.45, 0.6 or 0.9 mg of 17β-estradiol. In the case of a transdermal patch, a preferred patch delivers to systemic circulation a daily dose of 17β-estradiol (or an HRT equivalent amount of a suitable estrogen) between about 0.15 mg to about 1.5 mg and more preferably between about 0.3 mg to about 0.9 mg. A specific patch might be worn for one week and deliver to the surface of the skin in that period of time an average daily dose of 0.3, 0.45, 0.6 or 0.9 mg of 17β-estradiol. Regardless of the foregoing, it is intended herein to use conventional amounts of estrogen since it is not the estrogen component, which is critical to the invention. Persons skilled in the art well understand required doses of continuous estrogen in HRT regimens.

Norgestimate (NGM) or norelgestromin (NGMN) are the progestogens utilized herein and are each known to the art of hormone replacement therapy. In fact, norgestimate is now used in at least one commercially available HRT product. The preferred progestogen is norelgestromin (17-d-norgestimate). Norelgestromin is the major metabolite of norgestimate in humans with 80% and higher of norgestimate being converted to norelgestromin in vivo. For this reason, inhibition of sulfatase enzyme activity which is demonstrated for norelgestromin is inferred to norgestimate. Of course, to obtain equivalent inhibition of sulfatase enzyme activity, it may be necessary to administer a greater dose of norgestimate as compared to any dose of norelgestromin.

The objectives of any HRT regimen are basically achieved by the administration of an estrogen, which in practice is usually 17β-estradiol. The NGM or NGMN is administered in conjunction with the estrogen not so much to treat the condition in question, but in part, to oppose the action of the estrogen on the endometrium. It has been observed that the long term administration of an estrogen which is unopposed by the administration of a NGM or NGMN leads to a substantial increase in the incidence of endometrial cancer. Thus, it is a first requirement for the HRT regimen herein that the NGM or NGMN be administered in an amount which is an effective endometrium protective amount.

According to the present invention, it is now an additional requirement that the NGM or NGMN be administered to post-menopausal or estrogen deficient females in need of hormone replacement therapy in an amount which is an effective breast protective amount. In one characterization of a breast protective amount of NGM or NGMN and assuming an endometrium protective amount, there is administered sufficient active compound to provide for, during a substantial portion of each day, a substantial supression of sulfatase activity, for example, of 50% or greater and preferably of 67% or greater and most preferably of 75% or greater. A substantial portion of a day is intended to mean a period of at least 4 hours, but within the invention might mean a period of at least 8 hours or 12 hours or even 24 hours.

In the case of a daily oral tablet, there is administered a preferred dose of norgestimate or norelgestromin between 30 µg to 500 µg and more preferably between 45 µg to 300 µg. Specific daily oral tablets might contain 45, 60, 90, 120, 180 or 250 µg of norgestimate or norelgestromin. In the case of a vaginal ring, a preferred ring delivers to systemic circulation a daily dose of norgestimate or norelgestromin between 20 µg to 300 µg and more preferrably between 30 µg to 180 µg. A specific vaginal ring might be inserted for one week and deliver to systemic circulation in that period of time an average daily dose of 25, 35, 50, 70, 100 or 140 µg of norgestimate or norelgestromin. In the case of a transdermal patch, a preferred patch delivers to systemic circulation a daily dose of norgestimate or norelgestromin between 20 µg to 300 µg and more preferrably between 30 µg to 180 µg. A specific patch might be worn for one week and deliver to systemic circulation in that period of time an average daily dose of 25,35, 50,70,100 or 140 µg of norgestimate or norelgestromin.

In Table 1, there are disclosed preferred oral daily HRT regimens according to the present invention containing norgestimate (NGM) or norelgestromin (NGMN).

**Table 1**

| Regimen # | Tablet Administration | Tablet 17β-estradiol content | Tablet progestogen content |
|---|---|---|---|
| 1 | continuous | 0.5 mg | 45 µg NGM or NGMN |
| 2 | continuous | 0.75 mg | 45 µg NGM or NGMN |
| 3 | continuous | 1.0 mg | 45 µg NGM or NGMN |
| 4 | continuous | 1.5 mg | 45 µg NGM or NGMN |
| 5 | continuous | 0.5 mg | 60 µg NGM or NGMN |
| 6 | continuous | 0.75 mg | 60 µg NGM or NGMN |
| 7 | continuous | 1.0 mg | 60 µg NGM or NGMN |
| 8 | continuous | 1.5 mg | 60 µg NGM or NGMN |
| 9 | continuous | 0.5 mg | 90 µg NGM or NGMN |
| 10 | continuous | 0.75 mg | 90 µg NGM or NGMN |
| 11 | continuous | 1.0 mg | 90 µg NGM or NGMN |
| 12 | continuous | 1.5 mg | 90 µg NGM or NGMN |
| 13 | continuous | 0.5 mg | 120 µg NGM or NGMN |
| 14 | continuous | 0.75 mg | 120 µg NGM or NGMN |
| 15 | continuous | 1.0 mg | 120 µg NGM or NGMN |
| 16 | continuous | 1.5mg | 120 µg NGM or NGMN |
| 17 | continuous | 0.5 mg | 180 µg NGM or NGMN |
| 18 | continuous | 0.75 mg | 180 µg NGM or NGMN |
| 19 | continuous | 1.0 mg | 180 µg NGM or NGMN |
| 20 | continuous | 1.5 mg | 180 µg NGM or NGMN |
| 21 | continuous | 0.5 mg | 250 µg NGM or NGMN |
| 22 | continuous | 0.75 mg | 250 µg NGM or NGMN |
| 23 | continuous | 1.0 mg | 250 µg NGM or NGMN |
| 24 | continuous | 1.5 mg | 250 µg NGM or NGMN |
| 25 | Continuous, | A: 1.0 mg | A: 90 µg NGM or |
| | alternating | B: 1.0 mg | NGMN |
| | 3 days tablet A | | B: 180 µg NGM or |
| | 3 days tablet B | | NGMN |

In Table 2, there are disclosed preferred transdermal HRT regimens or vaginal ring regimens using weekly patches or rings containing norgestimate (NGM) or norelgestromin (NGMN). The weekly patches or rings deliver to systemic circulation the reported average daily dose of NGM or NGMN.

**Table 2**

| Regimen # | Device administration | Device 17β-estradiol average daily dose | Device progestogen average daily dose |
|---|---|---|---|
| 26 | Continuous | 0.3 mg | 25 µg NGM or NGMN |
| 27 | Continuous | 0.45 mg | 25 µg NGM or NGMN |
| 28 | Continuous | 0.6 mg | 25 µg NGM or NGMN |
| 29 | Continuous | 0.9 mg | 25 µg NGM or NGMN |
| 30 | Continuous | 0.3 mg | 35 µg NGM or NGMN |
| 31 | Continuous | 0.45 mg | 35 µg NGM or NGMN |
| 32 | Continuous | 0.6 mg | 35 µg NGM or NGMN |
| 33 | Continuous | 0.9 mg | 35 µg NGM or NGMN |
| 34 | Continuous | 0.3 mg | 50 µg NGM or NGMN |
| 35 | Continuous | 0.45 mg | 50 µg GM or NGMN |
| 36 | Continuous | 0.6 mg | 50 µg NGM or NGMN |
| 37 | Continuous | 0.9 mg | 50 µg NGM or NGMN |
| 38 | Continuous | 0.3 mg | 70 µg NGM or NGMN |
| 39 | Continuous | 0.45 mg | 70 µg NGM or NGMN |
| 40 | Continuous | 0.6 mg | 70 µg NGM or NGMN |
| 41 | Continuous | 0.9 mg | 70 µg NGM or NGMN |
| 42 | Continuous | 0.3 mg | 100 µg NGM or NGMN |
| 43 | Continuous | 0.45 mg | 100 µg NGM or NGMN |
| 44 | Continuous | 0.6 mg | 100 µg NGM or NGMN |
| 45 | Continuous | 0.9 mg | 100 µg NGM or NGMN |
| 46 | Continuous | 0.3 mg | 140 µg NGM or NGMN |
| 47 | Continuous | 0.45 mg | 140 µg NGM or NGMN |
| 48 | Continuous | 0.6 mg | 140 µg NGM or NGMN |
| 49 | Continuous | 0.9 mg | 140 µg NGM or NGMN |
| 50 | Continuous, | A: 0.6 mg | A: 50 mg |
| | alternating device A | B: 0.6 mg | B: 100 mg |
| | and device B | | |

The estrogen, and NGM or NGMN are orally administered preferably together in tablets also containing a pharmaceutically acceptable non-toxic carrier, but they can also be administered separately. Suitable carriers include magnesium carbonate, magnesium stearate, talc, lactose, sugar, peptin, dextrin, starch, methylcellulose, sodium carboxylmethylcellulose, and the like. The tablet may also contain one or more substances, which act as diluents, flavoring agents, solubilizers, lubricants, suspending agents, binders, or tablet disintegrating agents as well as encapsulating materials. In general, the active agents are processed, together with the usual additives, vehicles and/or flavor-ameliorating agents normally employed in Galenic pharmacy, in accordance with generally accepted pharmaceutical practices. The hormone containing tablets might also contain nutritional supplements such as, for example, iron supplements, folic acid, calcium, vitamin B₆, vitamin B₁₂, etc. In the manufacture of a typical tablet, the active agents are granulated with spray dried lactose, a lubricating agent and a colorant and compressed.

Oral tablets are preferably packaged in the form of a pharmaceutical kit or package in which the daily dosages are arranged for proper sequential administration. This invention also relates, therefore, to a pharmaceutical unit which contains the tablets of the regimen in a synchronized, fixed sequence, wherein the sequence or arrangement of the dosage units corresponds to the regimen of daily administration.

The estrogen and NGM or NGMN may be transdermally administered together by use of a patch. Broadly, patches are devices which contain at a minimum a drug reservoir matrix for holding the drug and metering the drug deposition or delivery to the skin, a backing, and an adhesive layer for adhering the device to the patient. The device may contain other layers such as a drug release rate controlling layer for modulating delivery rate, and the like. The device may contain permeation enhancers to increase the rate of penetration of drugs across the skin. Patches are well known and understood by persons skilled in the art. Patches are now employed in marketed products for the administration of certain progestogens and for the administration of 17β-estradiol specifically. Specific patches and even their application to steroids of the type described herein are described in U.S. Pat. Nos. 5474783; 5656286; 5958446; 6024976; 5252334; 5006342; and 4906463.

The estrogen and NGM or NGMN may be intravaginally administered together by use of a ring. Broadly, rings are devices having an elastomeric portion or body into which the active steroid is dispersed and which acts as a reservoir and meter for the diffusion of active to the lining of the vagina. The ring may be composed entirely of elastomer with steroid homogenously dispersed throughout as described in US Pat. No. 3545397. The ring may have an inert inner core surrounded by an active containing elastomeric layer as described in US Pat. No. 4012496. The ring may have an elastomeric active containing inner core surrounded by a thin elastomeric layer initially containing no active. The ring may have an inert core, surrounded by an active containing elastomeric layer and further surrounded by an elastomeric outer layer of variable thickness initially containing no active as described in US Pat. No. 4292965. The elastomer, the layered design of the ring, its surface area, the concentration of active, the nature of the active, etc. all combine to determine the release rate of active. Rings are well known and understood by persons skilled in the art. Rings are now employed in marketed products for the administration of certain steroids. Further specific rings and their application to steroids of the type described herein are described in U.S. Pat. Nos 4871543 and 5188835.

### BIOLOGICAL TEST METHODS

### Chemicals

[6,7⁻³H(N)]-estrone sulfate (³H-E₁S), ammonium salt (sp. act. 53 Ci/mmol) and [4-¹⁴C]-estradiol (¹⁴C-E₂) (sp. act. 57 mCi/mmol) were purchased from New England Nuclear Division (DuPont de Nemours, Les Ulls, France). The purity of the radioisotopes was assessed by thin-layer chromatography (TLC) in the appropriate system before use. E₁S, ammonium salt, unlabeled E₁ and E₂, (analytical grade) were obtained from Sigma-Aldrich Chimle, (St Quentin Fallavier, France). 17-deacetylnorgestimate (NGMN; 13-ethyl-17-hydroxy-18,19-dinor-17α-pregn-4-en-20-yn-3-one oxime) was a gift from R. W. Johnson Pharmaceutical Research Institute, Medicinal Chemistry Department, (Raritan, NJ, USA); medroxyprogesterone acetate (MPA, 17α-acetoxy-6α-methylprogesterone) was obtained from Sigma-Aldrich Chimie. All other chemicals were of the highest grade commercially available.

### Cell culture

The hormone-dependent MCF-7 and T-47D human mammary cancer cell lines were grown in Eagle's Minimal Essential Medium (MEM) buffered with 10 mmol/l HEPES (pH 7.6), supplemented with 2 mmol/l L-glutamine, 100 U/ml penicillin-streptomycin and 5% fetal calf serum (FCS) (A.T.G.C., Marne-la-Vallée, France) for T-47D, or 10% FCS for MCF-7 cells, and incubated at 37°C n a humidified atmosphere of 5% CO₂. Media were changed twice a week. The cells were passed every 10-12 days and replated in 75 cm² flasks (A.T.G.C.) at 3 x 10⁶ cells/flask. Four days before the experiments, the cells were transferred to MEM containing 5% steroid-depleted treated FCS. The FCS had been treated overnight at 4 °C with dextran-coated charcoal (DCC)(0.1-1% w/v, DCC-FCS). The MCF-7 and T-47D cell lines used herein were deposited in accordance with the Budapest Treaty under the references MCF7_JJPRD and T47D_JJPRD on May 17, 2002 at The Belgian Co-ordinated Collections of Microorganisms (BCCM), Laboratorium voor Moleculaire Biologie, Universiteit Gent, K. L. Ledeganckstraat 35, B-9000 Gent, Belgium and are publicly available under accession numbers LMBP 5862CB and LMBP 5863CB, respectively.

### Isolation and quantification of [³H]-estradiol from human mammary cancer cells incubated with [³H]-E₁S

Preconfluent cells were incubated for 4 hours at 37 °C in MEM-DCC-FCS with the addition of 5x10⁻⁹ mol/l of [³H]-E₁S, alone (control cells) or in combination with the different compounds: NGMN or MPA, dissolved in ethanol (final concentration < 0.2%), at a range of concentrations of 5x10⁻⁵ -5x10⁻⁹ mol/l. Control cells received ethanol vehicle only. After 24 hours, the medium was removed, the cells washed twice with ice-cold Hank's Buffered Saline Solution (HBSS, calcium-magnesium-free)(A.T.G.C.) and harvested by scraping. After centrifugation, the pallet was treated with 80% ethanol and the radioactivity extracted for at least 24h at -20°C. The cellular radioactivity uptake was determined in the ethanolic supernatant and the DNA content in the remaining pellet was evaluated according to Burton. *Biochem Journal 62:315-*323, *1956.* [¹⁴C]-E₂ (5,000 dpm) was added to monitor analytical losses and unlabeled E₁ and E₂ (50µg) were used as carriers and reference indicators. In the total ethanolic extracts, E₂ was isolated by thin layer chromatography (TLC) on silica gel 60F₂₅₄ (Merck, Darmstadt, Germany), developed with chloroform-ethylacetate (4:1, v/v) system. After visualization of the estrogens under U.V. at 254 nm, the appropriate areas were cut off into small pieces, placed in liquid scintillation vials with ethanol (0.5 ml) and allowed to extract for 30 mn. Three ml of Opti-fluor (Packard, Rungis, France) were added and the vials were analyzed for ³H and ¹⁴C contents with quench correction by external standarization. The quantitative evaluation of E₂ was calculated as a percentage of the total radioactivity associated with the cells and then expressed as fmol of E₂ formed /mg DNA from E₁S.

### Statistical analysis

Data are expressed as the mean ± standard error of the mean (SEM) values. Student's t-test was used to assess the significance of the differences between means; p values ≤ 0.05_were considered significant.

### RESULTS

Table 3 shows the effects of NGMN and medroxyprogesterone acetate (MPA) concentrations on the conversion of E₁S to E₂ in the hormone-dependent human breast cancer cell line T-47D The data are the mean ± SEM of duplicate determinations of 3 independent experiments. * p ≤ 0.05 vs contol values (non-treated cells); ** p ≤ 0.005 vs contol values (non-treated cells)

**TABLE 3**

| T-47D | | |
|---|---|---|
| NGMN or MPA conc 1x10⁻⁶ mol/l | NGMN E₂ formed fmol/mg DNA (% inhibition) | MPA E₂ formed fmol/mg DNA (% inhibition) |
| 0 (control) | 1805 ± 152(0%) | |
| 0.005 | 1029 ± ? (43 ± 7%)* | 1245 ± ? (31 ± 5%)* |
| 0.5 | 469 ± ? (74 ± 4%)* | 957 ±? (47 ± 3%)* |
| 50 | 54 ± ? (97 ± 2%)** | 704 ± ? (61 ± 3%)* |

Table 4 shows the effects of NGMN and medroxyprogesterone acetate (MPA) concentrations on the conversion of E₁S to E₂ in the hormone-dependent human breast cancer cell line MCF-7. The data are the mean ± SEM of duplicate determinations of 3 independent experiments. * p ≤ 0.05 vs contol values (non-treated cells); ** p ≤ 0.005 vs contol values (non-treated cells)

**TABLE 4**

| MCF-7 | | |
|---|---|---|
| NGMN or MPA conc 1 x 10⁻⁶ mol/l | NGMN E₂ formed fmol/mg DNA (% inhibition) | MPA E₂ formed fmol/mg DNA (% inhibition) |
| 0 / control | 2185 ± 101 (0%) | |
| 0.005 | 1639 ± ? (25 ± 4%)^{*} | 2054 ± ? (6 ± 3%) |
| 0.5 | 940 ± ? (57 ± 5%)* | 1748 ± ? (20 ± 3%) |
| 50 | 87 ± ? (96 ± 2%)** | 808 ± ? (63 ± 4%)* |

Table 5 shows the IC₅₀ values for NGMN and medroxyprogesterone acetate (MPA) in the conversion of E₁S to E₂ in the hormone-dependent human breast cancer cell lines MCF-7 and T-47D. IC₅₀ values correspond to the 50% inhibition of the conversion of E₁S to E₂ and were determined using non-linear regression analysis.

**TABLE 5**

| | IC₅₀, 1 x 10⁻⁶ mol/l | |
|---|---|---|
| | T-47D | MCF-7 |
| NGMN | 0.0127 | 0.178 |
| MPA | 2.15 | 26.1 |

## Claims

1. Use of a combination of estrogen, and norgestimate or norelgestromin for the manufacture of a medicament for use in a method of the prevention of breast cancer whilst providing hormone replacement therapy, which comprises the step of administering continuously to a post-menopausal or estrogen deficient female in need of such therapy, a combination of an effective hormone replacement amount of estrogen and of an effective endometrium protective amount and an effective breast protective amount of norgestimate or norelgestromin.

2. Use according to claim 1, wherein the step of administering continuously to a post-menopausal or estrogen deficient female in need of such therapy comprises a cycle of separate dosage units adapted for successive daily oral administration for the length of the cycle,

3. Use according to claim 1, wherein the step of administering continuously to a post-menopausal or estrogen deficient female in need of such therapy comprises or more transdermal patches adapted for successive administration for the length of the cycle,

4. Use according to claim 1, wherein step of administering continuously to a post-menopausal or estrogen deficient female in need of such therapy comprises one or more vaginal rings adapted for successive administration for the length of the cycle,

## Patentansprüche

1. Verwendung einer Kombination aus Östrogen und Norgestimat oder Norelgestromin zur Herstellung eines Medikaments zur Verwendung in einem Verfahren zur Verhütung von Brustkrebs während einer Hormonersatztherapie, welches den Schritt eines kontinuierlichen Verabreichens einer Kombination aus einer Menge an Östrogen, die als Hormonersatz wirksam ist, und einer Menge an Norgestimat oder Norelgestromin, die zum Schutz des Endometriums und der Brust wirksam ist, an eine Frau in der Postmenopause oder mit einem Östrogenmangel, die einer solchen Therapie bedarf, umfasst.

2. Verwendung nach Anspruch 1, wobei der Schritt des kontinuierlichen Verabreichens an eine Frau in der Postmenopause oder mit einem Östrogenmangel, die einer solchen Therapie bedarf, einen Zyklus aus getrennten Dosiereinheiten umfasst, die an eine aufeinanderfolgende tägliche orale Verabreichung über die Länge des Zyklus angepasst sind.

3. Verwendung nach Anspruch 1, wobei der Schritt des kontinuierlichen Verabreichens an eine Frau in der Postmenopause oder mit einem Östrogenmangel, die einer solchen Therapie bedarf, ein oder mehrere transdermale Pflaster umfasst, die an eine aufeinanderfolgende Verabreichung über die Länge des Zyklus angepasst sind.

4. Verwendung nach Anspruch 1, wobei der Schritt des kontinuierlichen Verabreichens an eine Frau in der Postmenopause oder mit einem Östrogenmangel, die einer solchen Therapie bedarf, einen oder mehrere Vaginalringe umfasst, die an die aufeinanderfolgende Verabreichung über die Länge des Zyklus angepasst sind.

## Revendications

1. Utilisation d'une association d'oestrogène, et de norgestimate ou de norelgestromine, pour fabriquer un médicament à utiliser dans un procédé de prévention du cancer du sein tout en fournissant un traitement hormonal substitutif, qui comprend l'étape consistant à administrer de manière continue, à une femme ménopausée ou présentant une déficience en oestrogène et ayant besoin d'un tel traitement, une association d'une quantité de substitution hormonale efficace d'oestrogène et d'une quantité de protection de l'endomètre efficace et d'une quantité de protection du sein efficace de norgestimate ou de norelgestromine.

2. Utilisation selon la revendication 1, dans laquelle l'étape consistant à administrer de manière continue, à une femme ménopausée ou présentant une déficience en oestrogène et ayant besoin d'un tel traitement, comprend un cycle d'unités d'administration séparées adaptées pour l'administration orale quotidienne successive pendant le cycle.

3. Utilisation selon la revendication 1, dans laquelle l'étape consistant à administrer de manière continue, à une femme ménopausée ou présentant une déficience en oestrogène et ayant besoin d'un tel traitement, comprend un ou plusieurs patchs adaptés pour l'administration successive pendant le cycle.

4. Utilisation selon la revendication 1, dans laquelle l'étape consistant à administrer de manière continue, à une femme ménopausée ou présentant une déficience en oestrogène et ayant besoin d'un tel traitement, comprend un ou plusieurs anneaux vaginaux adaptés pour l'administration successive pendant le cycle.
